# EUROPÄISCHE PATENTSCHRIFT

(11) **EP 1 044 702 B1**
(45) Veröffentlichungstag und Bekanntmachung des Hinweises auf die Patenterteilung: **01.06.2005**
(21) Anmeldenummer: 00104191.2
(22) Anmeldetag: 01.03.2000
(51) Int. Cl.: A61N 2/06, G01R 33/383, H01F 7/02

(54) **Magnetspirale**
Spiral-wound magnetic foil
Bande magnétisée enroulée en forme de spirale

(30) Priorität: 15.04.1999 DE 29906661 U
(43) Veröffentlichungstag der Anmeldung: 18.10.2000
(73) Patentinhaber: Rheinmagnet Horst Baermann GmbH, 53819 Neunkirchen-Seelscheid (DE)
(72) Erfinder: Baermann, Horst, 51429 Bergisch Gladbach (DE); Geissler, Horst, 50259 Pulheim (DE)
(74) Vertreter: Lippert, Stachow, & Partner

(56) Entgegenhaltungen:
- EP-A- 0 198 958
- WO-A-93/13720
- DE-A- 3 331 061
- DE-A- 3 719 306
- DE-A- 3 730 077
- DE-U- 9 412 807
- GB-A- 2 294 120

## Beschreibung

Die Erfindung betrifft eine Vorrichtung zur therapeutischen Anwendung mit einer Magnetspirale, die aus wenigstens einer Magnetfolie aus einem gummiartig-flexiblen Kunststoff besteht, wobei die Vorrichtung eine Wirkfläche aufweist, mit der das therapeutisch zu behandelnde Körperteil beaufschlagbar ist, und entlang der Wirkfläche der Vorrichtung eine Stirnseite der Magnetspirale angeordnet ist.

Die Verwendung von Magnetfeldern zur therapeutischen Anwendung ist hinlänglich bekannt. Beispielsweise zeigt die Druckschrift DE 3331061 A1 eine biegsame magnetische Folie für therapeutische Zwecke. Die therapeutische Wirkung beruht insbesondere auf dem Hall-Effekt, der eine Ladungstrennung in einem durch das Magnetfeld strömenden Elektrolyten bewirkt. Dabei werden elektrische Spannungen, welche die gewünschten therapeutischen Wirkungen in so behandelten Körperteilen hervorrufen können, quer zur Strömungsrichtung aufgebaut.

Hergestellt werden derartige Vorrichtungen zur therapeutischen Anwendung unter Verwendung von Folien aus einem gummiartig-flexiblen, vorzugsweise hautverträglichen Kunststoff. In den Kunststoff sind dauermagnetische Teilchen, vorzugsweise dauermagnetische Teilchen aus einem Ferrit- oder Seltenerdwerkstoff, wie beispielsweise Barium- oder Strontium-Ferrit bzw. NdFeB, eingebettet. Diese werden durch das Anlegen eines äußeren Magnetfeldes an eine oder beide Seiten der Folie ausgerichtet und erzeugen so ein magnetisch polarisiertes Gebiet, wobei die Folie als flächiges Gebilde mit einer handelsüblichen Folienstärke zwischen 0,3 mm und 1,5 mm vorliegt.

Aus dem Stand der Technik wie beispielsweise WO/93/13720 sind derartige Folien in verschiedenen Ausführungsformen bekannt. Sie werden durch Ausstanzen oder Ausschneiden aus handelsüblichen Magnetfolien gewonnen und liegen im allgemeinen in Form von runden Scheiben oder Rechtecken vor. Die Magnetisierung erfolgt in aller Regel einseitig lateral, zwei- oder mehrpolig. Dabei liegen die Polkonfigurationen üblicherweise in Form von geraden Streifen, konzentrischen Ringen, Spiralen, Rechtecken, Sektoren sowie anderen geometrischen Gebilden vor. Lediglich bei axialer Magnetisierung, bei der die eine Fläche einen einzigen N-Pol und die Rückseite der Folie einen einzigen S-Pol aufweist, weisen beide Seiten an vergleichbaren Meßpunkten gleiche magnetische Induktionswerte auf.

Die vorgenannte einseitige lateral-mehrpolige Magnetisierung weist gegenüber einer zweiseitig lateral-mehrpoligen Magnetisierung den Vorteil auf, dass sie allgemein sehr praktisch ist und auf der dem Körper zugewandten Folienseite höhere Induktionswerte aufweist als bei zweiseitig lateraler Polarisierung bei jeweils derselben Polkonfiguration, welche dies auch immer sei.

Nachteilig wirken sich die vorgenannten zwei- oder mehrpolig lateralen Magnetisierungen dieser flächigen Gebilde allerdings dadurch aus, dass der Verlauf des magnetischen Flusses im Inneren der Folien in großen Bereichen bogenförmig verläuft. Dadurch wird die an sich erwünschte vollständige Sättigungspolarisierung des gesamten zur Verfügung stehenden Magnetwerkstoffes auf der dem Körper zugewandten Nutzseite der Folie nicht mehr gewährleistet. Dies stellt sich sodann insbesondere bei solchen therapeutisch verwendeten Magnetfolien als Nachteil heraus, bei denen die Polbreite größer ist als die Folienstärke. Magnetisierungen mit Polbreiten, die mehr als das 1-fache, vielfach sogar das 20-fache der Folienstärke betragen, sind jedoch wegen ihrer großen Streuung in tiefere Körperbereiche erwünscht. Als zusätzlicher Nachteil dieser Magnetisierungsart hat sich ferner der Streufluss herausgestellt, der sich aufgrund der geringen relativen Permeabilität von µr ≈1,0 auf der Folienrückseite bildet. Dieser kann bis zu etwa 75 % des auf der dem Körper zugewandten Folienfläche betragen und vermindert den erwünschten Nutzfluss erheblich. In einigen Fällen wird dieser Nachteil jedoch dadurch ausgeglichen, dass die dem Körper abgewandte Folienseite mit einer magnetischen hochpermeablen Folie, z. B. aus dünnem, kohlenstoffarmem Stahlblech abgedeckt wird. Durch diesen magnetischen Rückfluß wird der Nutzfluss stark erhöht. Nachteilig ist hierbei jedoch die Verwendung von Stahlblech, das wegen seiner geringen Dicke erhebliche Verletzungsgefahren bedeutet, die Herstellung verteuert und die Flexibilität stark herabsetzt.

Zusammenfassend wird also der Einsatz solcher magnetotherapeutischer Vorrichtungen durch die Stärke der Polarisation beschränkt. Bei einer schwachen Polarisation der verwendeten Folie ist auch die durch das Magnetfeld im Körper erzeugte Wirkung schwach.

Eine spiralförmige Wicklung eines flexiblen permanentmagnetischen Bandes auf einen rohrförmigen Grundkörper ist aus der DE 37 19 306 A1 bekannt. Die Magnetspirale dient zur Verwendung für einen NMR-Tomographen. Das auf den Grundkörper aufgewickelte permanentmagnetische Band ist vor dem Abwickeln so magnetisiert worden, dass sich im Inneren des Grundkörpers ein quer zu dessen Achse gerichtetes homogenes Magnetfeld ergibt. Um die Homogenität zu erreichen, ist es notwendig, dass das permanentmagnetische Band eine im Verlauf seiner Länge wechselnde Magnetisierungsrichtung aufweist, wobei der Magnetisierungsvektor aufeinander folgende Drehungen von 180° beschreibt. Eine derartige Magnetisierung ist nicht nur sehr aufwändig. Sie ist darüber hinaus nicht zur therapeutischen Anwendung geeignet, wenn nicht das Innere des rohrförmigen Grundkörpers, sondern die Stirnseite der Spirale als die dem Körper zugewandte Wirkseite benutzt werden soll.

Der Erfindung liegt die Aufgabe zugrunde, eine Vorrichtung mit einer Magnetspirale zur therapeutischen Anwendung mit einer ihrer Stirnseiten als Wirkseite bereitzustellen, von der bei möglichst einfacher Herstellung ein starkes und wirkungsvolles Magnetfeld ausgeht.

Die Aufgabe wird erfindungsgemäß durch eine Vorrichtung der eingangs genannten Art gelöst, wobei die Magnetfolie vor dem Aufwickeln zu einer Spirale ausschließlich in axialer Richtung, d. h. senkrecht zu ihren größeren Seitenflächen, magnetisch polarisiert ist. Im aufgewickelten Zustand der Folie weist so die eine der größeren Seitenflächen zu der anderen größeren Seitenfläche der darüber gelagerten Wicklung hin. Vorzugsweise wird die magnetische Polarisation bis zur vollständigen Sättigung vorgenommen. Hierdurch entsteht im Querschnitt der Magnetspirale vom Zentrum aus in radialer Richtung eine Abfolge von unterschiedlichen magnetischen Polarisationen. Dieses Hintereinanderanordnen der magnetischen Polarisationen bewirkt, dass diese ein besonders starkes magnetisches Feld in der Umgebung der Stirnseiten der Magnetspirale erzeugen. Insbesondere wird die Stärke des Magnetfelds dadurch hervorgerufen, dass eine Vielzahl von magnetischen Dipolen hintereinander entlang ihrer Polachsen angeordnet sind. Bei der erfindungsgemäßen Magnetspirale kann die Folie um sich selbst oder um einen beliebig geformten Gegenstand im Zentrum der Spirale herum aufgewickelt sein. Die erfindungsgemäße Magnetspirale kann beispielsweise einen im wesentlichen kreisförmigen Umfang oder einen rechteckigen Umfang mit abgerundeten Ecken aufweisen.

Die Folie kann über ihre gesamte Länge auf einer ihrer größeren Seitenflächen einen einheitlichen Pol und auf der anderen größeren Seitenfläche den dazu entgegengesetzten Pol aufweisen. Andererseits können sich in Längsrichtung der Folie mehrere Bereiche mit unterschiedlicher Polarisation auf einer Seitenfläche anschließen. Bei einer Hintereinanderanordnung in Bezug auf die Längsrichtung unterschiedlicher Polarisationsbereiche auf einer Seitenfläche kommt es dazu, dass im aufgewickelten Zustand der Folie in einem Abschnitt zwei Bereiche gleicher Polarisation benachbart zueinander liegen. Während bei einer einheitlichen Polarisation der größeren Seitenflächen stets zwei unterschiedliche Polarisationen aneinanderstoßen, so dass im Querschnitt der Magnetspirale stets Nordpol und Südpol eines Dipols aneinanderstoßen, stößt bei einer unterschiedlichen Polarisation auf der großen Seitenfläche im Querschnitt der Magnetspirale mindestens einmal ein Nordpol eines Folienteils an einen Nordpol eines daran in Wicklungsrichtung liegenden, nachfolgenden Folienelements. Im Hinblick auf die Magnetisierung der Spirale entstehen in diesem Bereich, in dem beispielsweise zwei Gebiete mit einer gleichen Polarisation aufeinandertreffen, drei ringförmige Pole der Magnetspirale. Wenn ein solches Aufeinandertreffen gleicher Polarisationen einmal auftritt, so weist die Magnetspirale eine im wesentlichen rotationssymmetrische Polarisation auf, die sich in radialer Richtung beispielsweise mit einem Nordpol, Südpol und wieder Nordpol ergibt. Die Polabfolge ergibt sich daraus, dass zwei Folienelemente, bei denen beispielesweise jeweils Nordpol an Nordpol stößt, entgegengesetzt zueinander ausgerichtet sind. Zur therapeutischen Anwendung kann auch ein solches magnetisches Feld mit mehreren Polen vorteilhaft sein, da der Wechsel der magnetischen Felder zusätzliche und im besonderen Maße erwünschte Wirkungen auf vorbeiströmende Elektrolyte hat.

Vorzugsweise haben die Bereiche gleicher Polarisation in Längsrichtung der Folie eine Länge, die mindestens einem Winkel von 2 π, d. h. einer Umwicklung, entspricht, so dass in der Fläche eines Querschnitts durch die Magnetspirale senkrecht zu den größeren Seitenflächen der Folie eine abwechselnde Folge von mindestens zwei ringförmigen Polen unterschiedlicher Vorzeichen vorliegt. Hierdurch wird sichergestellt, dass das in der Fläche eines Querschnitts durch die Magnetspirale im wesentlichen radiale Magnetfeld rotationsymmetrisch bezüglich des Zentrums der Magnetspirale ist.

Die Folie weist zweckmäßigerweise die Form eines länglichen Streifens mit rechtwinkligem Querschnitt auf, wobei in möglichst gleichförmiger statistischer Verteilung pulverförmige Magnetwerkstoffe, vorzugsweise Sr-Ferrit oder NdFeB mit Korngrößen kleiner als 1 mm, besonders bevorzugt kleiner als 100 µm, eingebettet sind. Bei einem solchen Streifen sind die größeren Seitenflächen diejenigen Flächen, die in Längsrichtung des Streifens verlaufen und breiter sind. Eine streifenförmige Folie lässt sich besonders leicht aufwickeln. Zur Herstellung einer erfindungsgemäßen Magnetspirale ist es für die praktische Verwendung sinnvoll, im aufgewickelten Zustand das freie Streifenende an der Magnetspirale zu befestigen. Dies kann beispielsweise durch die Verwendung eines Klebestreifens oder durch ein sonstiges Haltemittel geschehen.

In einer bevorzugten Weiterentwicklung der Erfindung sind auf jeder der größeren Seitenflächen in Querrichtung der Folie mindestens zwei unterschiedliche Polarisationen aufgebracht, wobei die Polarisation jeweils entgegengesetzt ist. Hierdurch entsteht neben der magnetischen Wirkung durch die axiale Polarisation der Folie zusätzlich durch die magnetische Polarisation auf der größeren Seitenfläche, die gleichsam wi bei einem quer zur axialen Richtung der Folie angeordneten Dipol wirkt, eine weitere Quelle für das magnetische Feld. So kann die Feldverteilung der Magnetspirale vorteilhaft modifiziert werden.

Die Magnetspirale kann, wie schon vorstehend erwähnt, auch in Längsrichtung der Folie mehrere aneinander anschließende Bereiche mit unterschiedlicher Polarisation auf den beiden größeren Seitenflächen aufweisen.

In einem weiteren Ausführungsbeispiel der Erfindung sind mindestens zwei Folien hintereinander zu einer Spirale aufgewikkelt, wobei die Polarisation der größeren Seitenflächen beim Übergang von einer Folie zu der sich anschließenden Folie wechselt.

Die Magnetspirale kann eine im wesentlichen zylindrische Form aufweisen, deren Zylinderhöhe kleiner als der Zylinderdurchmesser ist.

Die erfindungsgemäße Aufgabe wird ebenfalls gelöst durch eine Vorrichtung zur therapeutischen Anwendung, insbesondere eine Bandage, Tasche und Gesichtsmaske, die mindestens eine oben beschriebene Magnetspirale enthält. Mindestens eine zu einer Spirale aufgewickelte Folie ist zur therapeutischen Anwendung in einer Vorrichtung angeordnet. Hierfür eignen sich beispielsweise Bandagen, Taschen und Gesichtsmasken. Die Vorrichtung wird mit ihrer Wirkfläche bezüglich des zu behandelnden Körperteils möglichst nahe an dem Körperteil, das therapeutisch behandelt werden soll, getragen.

In einer Fortführung der Erfindung sind mindestens zwei jeweils zu einer Spirale aufgewickelte Folien, beabstandet voneinander, vorgesehen. Hierdurch kann, gleichsam wie bei einem Helmholtz-Spulenpaar, zwischen den beiden Spiralen ein Magnetfeld erzeugt werden. Ein solches Magnetfeld kann für eine therapeutische Anwendung der vorrichtung besonders vorteilhaft sein. Auch können die einzelnen zu Spiralen aufgewickelten Folien in der Vorrichtung so angeordnet werden, dass ganz besondere Feldkonfigurationen entstehen, sofern dies für eine therapeutische Wirkung gewünscht wird.

In einer bevorzugten Ausführung der Erfindung sind mindestens zwei zu jeweils einer Spirale aufgewickelte Folien stirnseitig im wesentlichen mit aufeinander fallenden Spiralachsen und auf Abstand zueinander vorgesehen. Hierdurch entstehen beispielsweise wenigstens zwei alternierende Magnetpole, zwischen denen der zu behandelnde Körperteil angeordnet werden kann.

Zweckmäßigerweise ist die Stirnseite der Spirale in der Umgebung des zu behandelnden Körperteils angeordnet. Vorteilhaft an einer solchen Anordnung der Spirale ist, dass das Magnetfeld entlang der Wirkfläche der Vorrichtung besonders stark vorhanden ist. So kann beispielsweise die Spirale in einer Bandage oder einer Tasche parallel zur Haut angeordnet werden.

Die erfindungsgemäße Magnetspirale soll nachfolgend anhand von Ausführungsbeispielen näher erläutert werden. Die dazugehörigen Zeichnungen zeigen in
- Fig. 1a: eine Magnetspirale,
- Fig. 1b: zwei weitere mögliche Formbeispiele für Magnetspiralen,
- Fig. 2: eine Magnetfolie mit axialer Magnetisierung,
- Fig. 3: eine Magnetfolie mit mehreren aneinander anschließenden Bereichen unterschiedlicher Polarisation,
- Fig. 4: eine Magnetspirale mit einem außen liegenden Nordpol und einem innen liegenden Südpol aus/mit einer Magnetfolie gemäß Fig. 2,
- Fig. 5: eine Magnetspirale mit einer alternierenden radialen Polfolge aus einer Magnetfolie gemäß Fig. 3,
- Fig. 6: eine Folie mit axialer Polarisation auf den größeren Seitenflächen,
- Fig. 7a: eine Magnetfolie mit zwei unterschiedlichen Polarisationen auf den größeren Seitenflächen,
- Fig. 7b: eine Folie mit mehreren unterschiedlichen Polarisationen auf den größeren Seitenflächen,
- Fig. 8: Induktionswerte an einer Magnetspirale abhängig von Dicke und Abstand mit einer Magnetspirale gemäß Fig. 4 gemessen,
- Fig. 9: Messungen des Abstandes zweier axial zueinander angeordneten Magnetspiralen gemäß Figur 4, deren Pole entgegengesetzt zueinander polarisiert sind, bei konstanter Feldstärke im Zentrum der Anordnung,
- Fig. 10: Anordnung der Magnetspiralen zur Messung des Abstandes bei konstanter Feldstärke gemäß Figur 9 und
- Fig. 11: ein Paar von Magnetspiralen im Querschnitt, die koaxial und beabstandet voneinander mit entgegengesetzten Polen angeordnet sind.

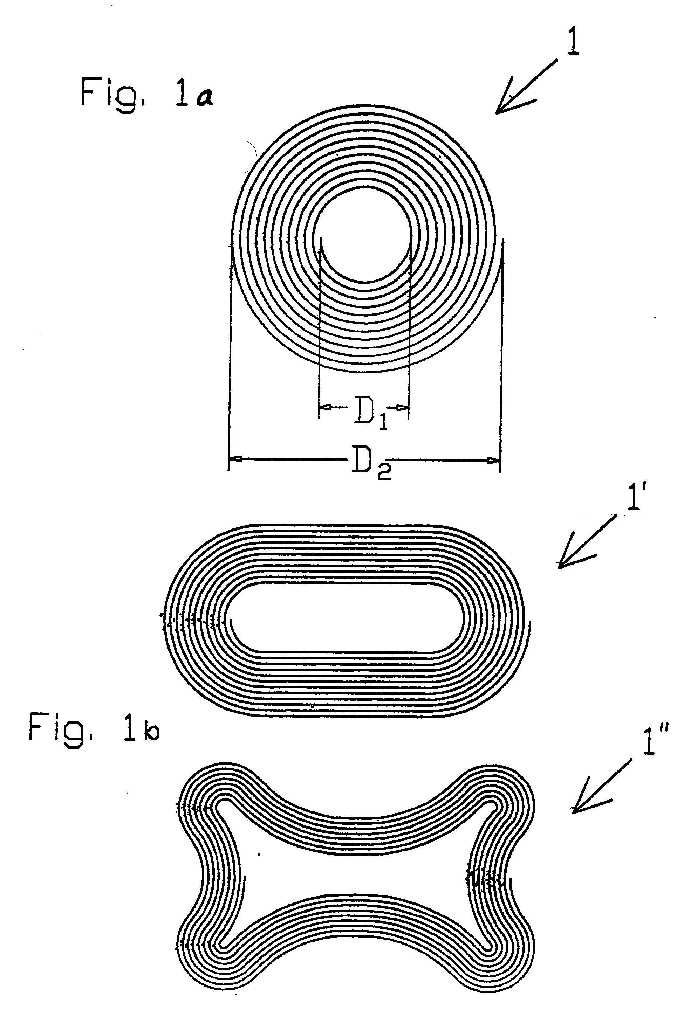

Die Magnetspirale 1 mit kreisförmigen Umfang weist einen Innendurchmesser D1 auf. In der in Figur 1a dargestellten Magnetspirale 1 ist eine Folie bis zu einem Außendurchmesser D2 aufgewickelt. Hierbei verbleibt ein im wesentlichen kreisförmiger Innenbereich mit dem Durchmesser D1 im Zentrum der Magnetspirale 1 frei. In diesem Innenbereich können Gegenstände angeordnet werden. Abmessungen solcher Magnetspiralen können sein: D1= 3 mm, D2= 40 mm, Höhe: 2,5 mm; D1= 5 mm, D2= 150 mm, Höhe: 15 mm; D1= 50 mm, D2= 150 mm, Höhe: 30 mm.

Die gewählte Form einer kreisförmigen Spirale ist in Figur 1a beispielhaft gewählt. So kann die Spirale auch durch das Umwickeln um einen beispielsweise quadratischen Gegenstand die Form eines Quadrats mit abgerundeten Ecken annehmen oder unregelmäßige Formen 1', 1'' wie in Figur 1b dargestellt aufweisen.

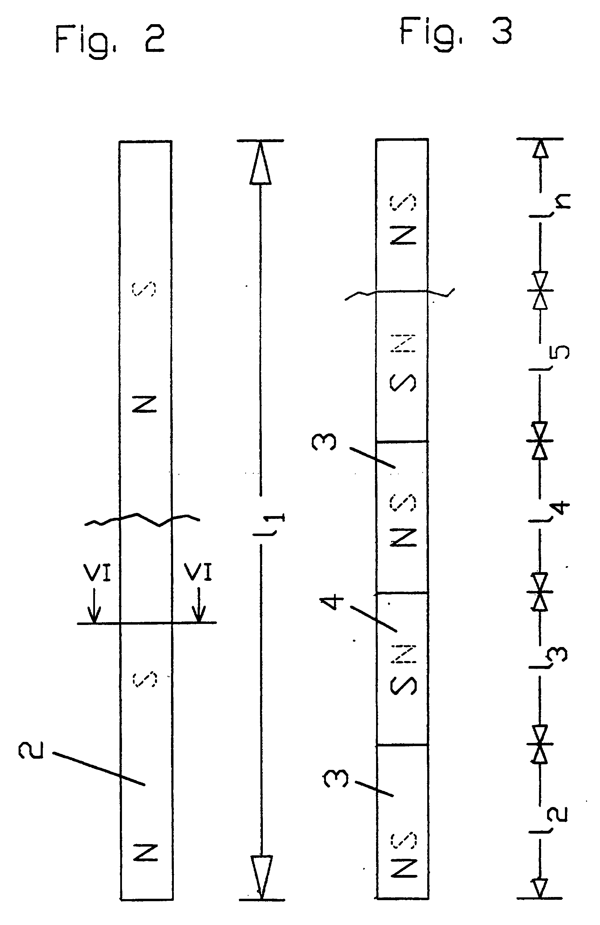

Die Folie, aus der die Magnetspirale durch Aufwickeln entsteht, weist in dem in Figur 2 dargestellten Ausführungsbeispiel eine einheitliche axiale magnetische Polarisierung, d. h. senkrecht zu ihren größeren Seitenflächen, auf. Die Folie besitzt die Länge L1 und kann entlang dieser aufgewikkelt werden.

Im Anschluss an die erste aufgewickelte Folie können weitere Folien um die erste Folie herumgewickelt werden. Dabei kann bei der zweiten Folie, die um die erste Folie gewickelt wird, die Polarisation der größeren Seitenfläche fortgesetzt oder entgegengesetzt sein. Eine solches Hintereinanderwickeln von mehreren Folien wirkt auf das Magnetfeld der Magnetspirale so wie eine einheitliche bzw. eine abwechselnde Polarisation in Längsrichtung bei der Verwendung genau einer Folie.

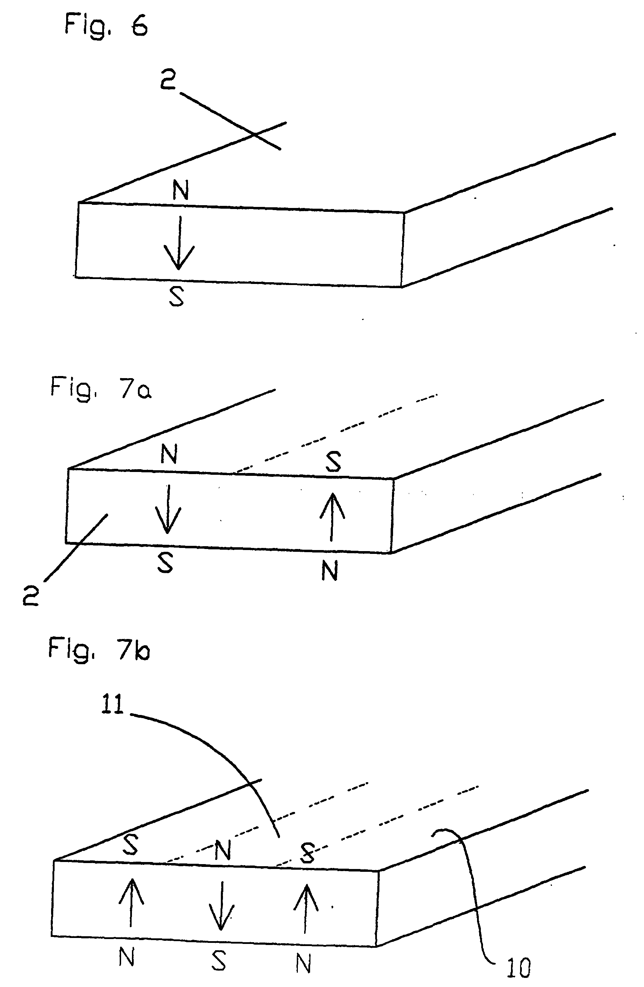

In Figur 2 ist dargestellt, dass die Rückseite (gestrichelt dargestellt) die entgegengesetzte Polarisation aufweist. Die dargestellte Folie ist mithin axial polarisiert, was auch Figur 6, in der ein Querschnitt durch einen Folienstreifen gezeigt ist, verdeutlicht.

Bei der in Figur 3 dargestellten Folie schließen auf den größeren Seitenflächen Bereiche 3 und 4 mit unterschiedlicher Polarisation aneinander an. Hierbei weist der Bereich 3 eine Länge L2 und der Bereich 4 eine Länge L3 auf. Die Längen sind hierbei so, dass im aufgewickelten Zustand der Folie jeweils mindestens eine Umwicklung erfolgt.

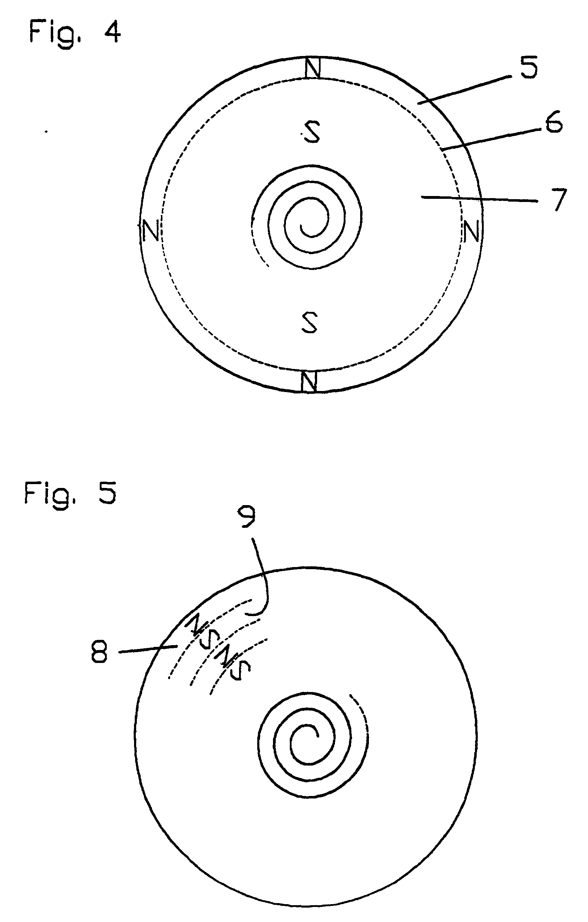

Ist die Folie 2 aus Figur 2 aufgewickelt, so ergibt sich eine Magnetspirale, die auf ihrer Stirnseite zwei Pole aufweist. wird die mit einem magnetischen Südpol magnetisierte Seitenfläche der Folie 2 innen liegend verwendet, so ergibt sich die in Figur 4 dargestellte Polverteilung der Magnetspirale. Ein magnetischer Nordpol 5 ist im peripheren Bereich der Magnetspirale und ein magnetischer Südpol 7 im zentralen Bereich der Magnetspirale angeordnet. Im Zwischenbereich läuft eine Trennlinie 6, die gleichsam den Übergang zwischen den magnetischen Polen 5 und 7, also eine neutrale Zone, markiert.

Werden, so wie in Figur 3 dargestellt, mehrere unterschiedlich magnetisch polarisierte Bereiche hintereinander angeordnet, so kann sich eine Polverteilung, wie in Figur 5 dargestellt, herausbilden. Hierbei wechseln sich magnetischer Nordpol 8 und magnetischer Südpol 9 ab.

Auch ist es möglich, auf den größeren Seitenflächen der Folie mehrere Bereiche magnetischer Polarisation aufzubringen. So zeigt Figur 6 einen Querschnitt der in Figur 2 dargestellten Magnetfolie mit einer einheitlichen Polarisation 2 auf der größeren Seitenfläche. Die in Figur 6 dargestellte Folie ist axial magnetisiert, denn Nord- und Südpol liegen einander auf der größeren Seitenfläche der Folie gegenüber.

Figuren 7a und 7b zeigen eine axial magnetisierte Folie, bei der mehrere quer angeordnete Bereiche unterschiedlicher Magnetisierung nebeneinander auf der größeren Seitenfläche aufgebracht sind. In Figur 7b grenzt der im Randbereich der Folie angeordnete Bereich 10 an einen zentralen Bereich 11 mit entgegengesetzter Polarisation.

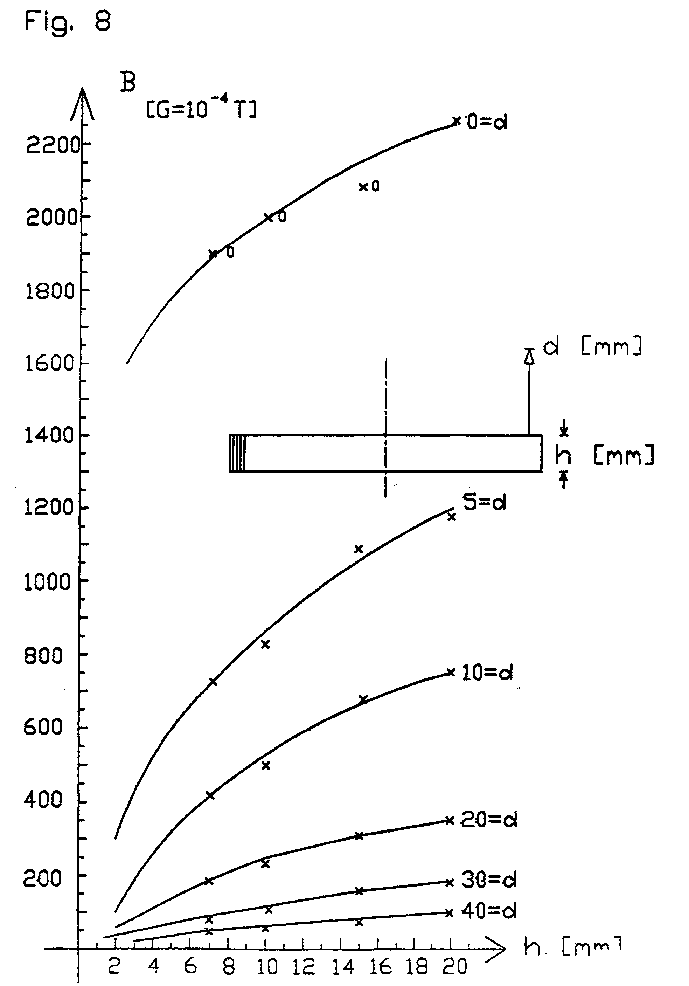

Beispielhaft wird die Wirkungsweise einer Magnetspirale anhand der nachfolgenden Messungen verdeutlicht. In Figur 8 ist die magnetische Flußdichte einer Magnetspirale in Abhängigkeit von deren Höhe h und dem Abstand d des Meßpunktes aufgetragen. Der Figur ist zu entnehmen, dass bei zunehmender Höhe h der Magnetspirale die magnetische Induktion zunimmt.

Ebenso ist den in Figur 8 dargestellten Messungen zu entnehmen, dass mit zunehmendem Abstand d von der Oberfläche der Magnetspirale die Stärke der magnetischen Flußdichte abnimmt. Der Abstand d ist hierbei jeweils in mm angegeben. Die gemessenen Werte zeigen ungefähr eine vier- bis zehnfache Stärke bei gleichem magnetischen Werkstoff im Vergleich zu der nicht aufgewickelten Verwendung der Folie. Hierdrin liegt ein wesentlicher Wirkungsvorteil der Magnetspirale.

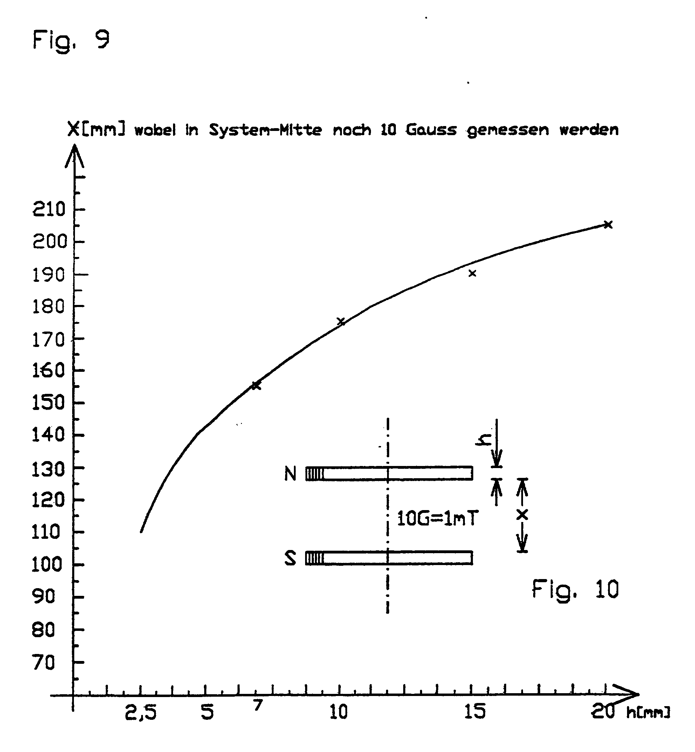

Figur 9 zeigt die Abhängigkeit des Abstandes x zweier parallel zueinander angeordneter Magnetspiralen mit entgegengesetzter Polarität von deren Höhe h, unter der Nebenbedingung, dass in der Mitte zwischen beiden Magnetspiralen aufgewickelt gemäß Figur 4 die magnetische Induktion 1 mT = 10 Gauß beträgt. Eine solche Anordnung ist in Figur 10 dargestellt. Diese Anordnung verdeutlicht, dass in dem Raumbereich zwischen zwei stirnseitig parallel zueinander angeordneten Magnetspiralen eine überaus starke und mithin wirkungsvolle magnetische Flußdichte erzielt werden kann, die sich zu einer therapeutischen Anwendung eignet. Eine solche Anordnung der Spiralen kann vorteilhaft beispielsweise in Knie-, Rücken-, Hüft-, Arm-, Ellenbogenbandagen verwendet werden, wobei die erfindungsgemäß erzielten hohen Induktionswerte bei den anwendungsgegebenen Abständen von den Oberflächen der Magnetspiralen von besonderem Vorteil sind.

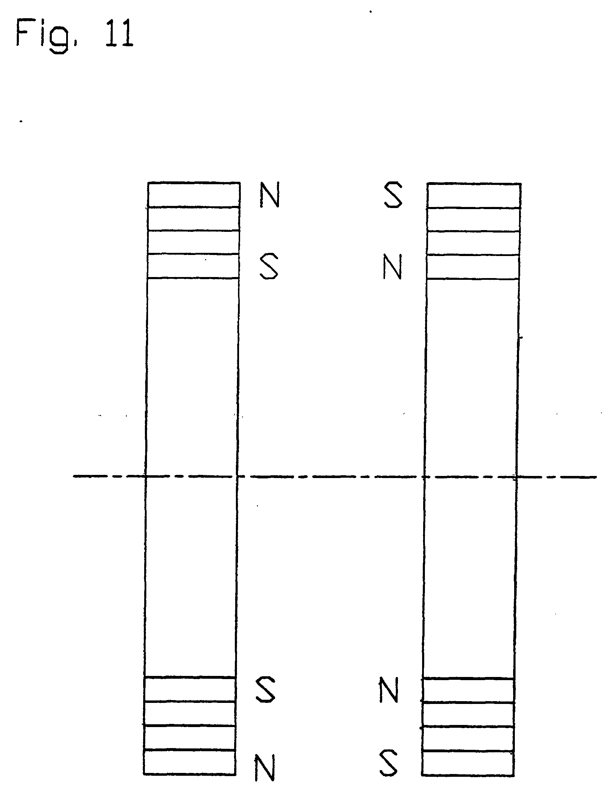

Eine weitere vorteilhafte Anordnung von zwei Magnetspiralen ist in Figur 11 im Querschnitt gezeigt. Hier liegen sich zwei Magnetspiralen so gegenüber, dass jeweils zwei entgegengesetzte Pole einander zugewandt sind. Mit einer solchen Anordnung der Magnetspiralen werden in dem zwischen den Magnetspiralen liegenden Volumen besonders hohe Induktionswerte erreicht, die für eine therapeutische Behandlung eines in diesem Bereich angeordneten Körperteils vorteilhaft sind.

### Bezugszeichenliste

- 1: Magnetspirale
- 2: Bereich magnetischer Polarisation (Nordpol)
- 3: Bereich magnetischer Polarisation (Nordpol)
- 4: Bereich magnetischer Polarisation (Südpol)
- 5: peripherer Nordpol der Magnetspirale
- 6: Trennlinie
- 7: zentraler magnetischer Südpol
- 8: peripherer magnetischer Nordpol
- 9: Südpol der Magnetspirale
- 10: Bereich magnetischer Polarisation (Südpol auf der Längsseite)
- 11: Bereich magnetischer Polarisation (Nordpol auf der Längsseite)

## Patentansprüche

1. Vorrichtung zur therapeutischen Anwendung mit einer Magnetspirale (1) die aus wenigstens einer Magnetfolie aus einem gummiartig-flexiblen Kunststoff besteht, wobei die Vorrichtung eine Wirkfläche aufweist, mit der das therapeutisch zu behandelnde Körperteil beaufschlagbar ist, und entlang der Wirkfläche der Vorrichtung eine Stirnseite der Magnetspirale angeordnet ist, **dadurch gekennzeichnet, dass** die Magnetfolie zu einer Spirale aufgewickelt ist, wobei die Magnetfolie vor dem Aufwickeln zu der Spirale ausschließlich axial, d.h. senkrecht zu ihren größeren Seitenflächen, magnetisch polarisiert ist und im aufgewickelten Zustand der Magnetfolie die eine der größeren Seitenflächen zu der anderen größeren Seitenfläche der darüber gelagerten Wicklung hin weist.

2. Vorrichtung nach Anspruch 1, **dadurch gekennzeichnet, dass** die Bereiche gleicher Polarisation in Längsrichtung der Magnetfolie eine Länge aufweisen, die mindestens einem Winkel von 2π, d.h. einer Umwicklung, entspricht, so dass in der Fläche eines Querschnittes durch die Magnetspirale(1) senkrecht zu den größeren Seitenflächen der Magnetfolie eine abwechselnde Folge von mindestens zwei ringförmigen Polen unterschiedlicher Vorzeichen vorliegt.

3. Vorrichtung nach Anspruch **1** oder 2, **dadurch gekennzeichnet, dass** auf jeder größeren Seitenfläche in Querrichtung der Magnetfolie mindestens zwei unterschiedliche Polarisationen (10, 11) aufgebracht sind, wobei diese Polarisation jeweils entgegengesetzt ist.

4. Vorrichtung nach einem der Ansprüche 1 bis 3, **dadurch gekennzeichnet, dass** in Längsrichtung die Magnetfolie sich mehrere Bereiche mit unterschiedlicher polarisation (3, 4)auf einer Seitenfläche anschließen.

5. Vorrichtung nach einem der Ansprüche 1 bis 4, **dadurch gekennzeichnet, dass** mindestens zwei Magnetfolien hintereinander zu einer spirale aufgewickelt sind, wobei die Polarisation der größeren Seitenflächen beim Übergang von einer Folie zu der sich anschließenden Folie wechselt.

6. Vorrichtung nach einem der Ansprüche 1 bis 5, **dadurch gekennzeichnet, dass** mindestens zwei jeweils zu einer Spirale aufgewickelte Magnetfolien beabstandet voneinander vorgesehen sind.

7. Vorrichtung nach einem der Ansprüche 1 bis 6, **dadurch gekennzeichnet, dass** sie zwei Magnetspiralen aufweist, die so angeordnet sind, dass sie beim Anlegen an das therapeutisch zu behandelnde Körperteil mit ihren den Wirkflächen der Vorrichtung zugeordneten Stirnseiten im wesentlichen mit aufeinanderfallenden Spiralachsen und im Abstand zueinander angeordnet sind und das Körperteil dazwischen einschließen.

8. Vorrichtung nach einem der Ansprüche 1 bis 7, **dadurch gekennzeichnet, dass** sie als Bandage ausgebildet ist.

9. Vorrichtung nach einem der Ansprüche 1 bis 7, **dadurch gekennzeichnet, dass** sie als Tasche ausgebildet ist.

10. Vorrichtung nach einem der Ansprüche 1 bis 7, **dadurch gekennzeichnet, dass** sie als Gesichtsmaske ausgebildet ist.

## Claims

1. Device for therapeutical application with a magnetic spiral (1) having at least one magnetic foil consisting of a rubber-type flexible plastic, said device having an active area for acting upon the part of the body to be therapeutically treated and a front face of said magnetic spiral being arranged along said active area of said device, **wherein** the magnetic foil is coiled up to a spiral the magnetic foil being magnetically polarized solely in axial direction i.e. vertically to its larger side faces prior to coiling it up and the one of the larger side faces facing the other larger side face of the winding arranged above in the coiled state.

2. Device according to claim 1, **wherein** the areas of identical polarization in lengthwise direction of the foil have a length, which is equal at least an angle of 2π, i.e. one winding, so that, in the area of a cross section through the magnetic spiral (1) vertically to the larger side faces of the foil, an alternating sequence of at least two ring-shaped poles of different polarity occur.

3. Device according to claim 1 or 2, **wherein** at least two varying polarizations (10, 11) are applied to each of the larger side face, whereby the polarization is in each case set in opposition.

4. Device according to one of the claims 1 to 3,
**wherein** several areas with varying polarization (3, 4) follow one another on a lateral face in lengthwise direction of the magnetic foil.

5. Device according to one of the claims 1 to 4,
**wherein** at least two foils are coiled up, one after the other into one spiral, whereby the polarization of the lager side faces changes at the transition from the one foil to the succeeding foil.

6. Device according to one of the claims 1 to 5,
**wherein** at least two foils, each coiled up into a spiral, are intended on the face side, essentially parallel to one another.

7. Device according to one of the claims 1 to 6,
**wherein** the device has two magnetic spirals arranged in such a way, that, in the case of applying at the part of the body to be therapeutic treated, the spirals are arranged with their front faces, which are assigned to the active areas, essentially with spiral axes positioned on top of one another and with a space between them and include the part of the body between them.

8. Device according to one of the claims 1 to 7,
**wherein** the device is designed as bandage.

9. Device according to one of the claims 1 to 7,
**wherein** the device is designed as pocket.

10. Device according to one of the claims 1 to 7,
**wherein** the device is designed as face mask.

## Revendications

1. Dispositif d'utilisation thérapeutique comportant une spirale magnétique (1), qui est constituée par au moins une feuille magnétique formée d'une matière plastique flexible type caoutchouc, le dispositif possédant une surface active, avec laquelle la partie du corps à traiter par voie thérapeutique peut être chargée, et une face frontale de la spirale magnétique est disposée le long de la surface active du dispositif, **caractérisé en ce que** la feuille magnétique est enroulée sous la forme d'une spirale, la feuille magnétique étant polarisée magnétiquement axialement, c'est-à-dire perpendiculairement à ses surfaces latérales plus grandes, avant l'enroulement pour former la spirale et que lorsque la feuille magnétique est à l'état enroulé, l'une des faces latérales les plus grandes est tournée en direction de l'autre des faces latérales les plus grandes de l'enroulement superposé.

2. Dispositif selon la revendication 1, **caractérisé en ce que** les zones de polarisation identique de la direction longitudinale de la feuille magnétique possèdent une longueur qui correspond au moins à un angle égal à 2π, c'est-à-dire un enveloppement, de sorte que dans la surface d'une section transversale de la spirale magnétique (1) perpendiculairement aux surfaces latérales plus grandes de la feuille magnétique, une succession alternée d'au moins deux pôles de forme annulaire ayant des signes différents est présente.

3. Dispositif selon la revendication 1 ou 2, **caractérisé en ce qu'**au moins deux polarisations différentes (10, 11) sont appliquées à chaque surface latérale plus grande sont appliquées dans la direction transversale de la feuille magnétique, ces polarisations étant réciproquement opposées.

4. Dispositif selon l'une des revendications 1 à 3, **caractérisé en ce que** dans la direction longitudinale, à la feuille magnétique (3, 4) se raccordent plusieurs zones ayant des polarisations différentes (3, 4) à une surface latérale.

5. Dispositif selon l'une des revendications 1 à 4, **caractérisé en ce qu'**au moins deux feuilles magnétiques sont enroulées l'une derrière l'autre pour former une spirale, la polarisation des surfaces latérales plus grandes alternant lors du passage d'une feuille à la feuille suivante.

6. Dispositif selon l'une des revendications 1 à 5, **caractérisé en ce qu'**au moins deux feuilles magnétiques enroulées respectivement pour former une spirale sont prévues à distance l'une de l'autre.

7. Dispositif selon l'une des revendications 1 à 6, **caractérisé en ce qu'**il comporte deux spirales magnétiques qui sont disposées de telle sorte que lors de leur application sur une partie du corps à traiter par voie thérapeutique, avec leurs faces frontales tournées de façon spécifique vers les surfaces actives du dispositif, ces spirales sont disposées avec leurs axes qui essentiellement se rencontrent et sont disposées à distance l'une de l'autre et enserrent entre elles la partie du corps.

8. Dispositif selon l'une des revendications 1 à 7, **caractérisé en ce qu'**il est agencé sous la forme d'un bandage.

9. Dispositif selon l'une des revendications 1 à 7, **caractérisé en ce qu'**il est agencé sous la forme d'une poche.

10. Dispositif selon l'une des revendications 1 à 7, **caractérisé en ce qu'**il est agencé sous la forme d'un masque placé sur le visage.
